# FASCICULE DE BREVET EUROPEEN

(11) **EP 0 438 332 B1**
(45) Date de publication et mention de la délivrance du brevet: **08.04.1998**
(21) Numéro de dépôt: 91400049.2
(22) Date de dépôt: 11.01.1991
(51) Int. Cl.: C07K 14/16, A61K 39/21, G01N 33/569

(54) **Peptides dérivant de la glycoprotéine d'enveloppe de virus HIV, leurs applications à la détection d'une infection due à ces virus et à la vaccination contre le SIDA**
Peptide, von Virus-HIV-Hüllen-Glycoproteinen abstammend, deren Verwendung zum Nachweis einer Infektion dieser Viren und für die Impfung gegen AIDS
Peptides issued from the glycoprotein envelope of HIV virus, their uses for the detection of an infection due to these viruses and for the vaccination against AIDS

(30) Priorité: 16.01.1990 FR 9000455; 16.01.1990 FR 9000456
(43) Date de publication de la demande: 24.07.1991
(73) Titulaire: ORGENICS LTD., Yavne 70 650 (IL)
(72) Inventeur: Martin, Jacques, F-75014 Paris (FR); Somme, Gérard, F-91140 Bures-Sur-Yvette (FR)
(74) Mandataire: Ores, Irène

(56) Documents cités:
- EP-A- 0 214 709
- EP-A- 0 247 557
- EP-A- 0 251 612
- EP-A- 0 278 148
- EP-A- 0 283 327
- EP-A- 0 284 383
- EP-A- 0 292 454
- EP-A- 0 326 490
- WO-A-86/06414
- WO-A-87/04459
- WO-A-87/06005
- WO-A-88/08005
- WO-A-89/03844
- PROCEEDINGS OF THE NATIONAL ACADEMY OF SCIENCES USA, vol. 85, juillet 1988, pages 5225-5229; P.J. KLASSE et al.: "Presence of antibodies to a putatively immunosuppressive part of human immunodeficiency virus (HIV) envelope glycoprotein gp41 is strongly associated with health among HIV-positive subjects"
- NATURE, vol. 326, 16 avril 1987, pages 662-669, Londres, GB; M. GUYADER et al.: "Genome organization and transactivation of the human immunodeficiency virus type 2"

## Description

La présente invention a pour objet des peptides antigéniques susceptibles d'être reconnus par des anticorps induits chez l'Homme par les rétrovirus du type HIV selon la nomenclature définie dans la revue Nature. L'invention concerne également des peptides ayant des propriétés immunogènes ou susceptibles d'être rendus immunogènes *in vivo*. L'invention concerne en outre des applications de ces peptides à la fabrication de composition pour le diagnostic *in vitro* chez l'Homme du SIDA et à la production de compositions immunogènes et de compositions vaccinantes contre les virus HIV.

De même l'invention concerne les applications aux mêmes fins des anticorps susceptibles d'être induits *in vivo* par les peptides immunogènes ou rendus immunogènes et, leurs applications à la production de principes actifs de médicaments contre certaines formes de SIDA.

L'invention concerne aussi l'utilisation de ces peptides dans des procédés de diagnostic *in vitro* de l'infection par les virus HIV-1 et/ou HIV-2, ainsi que les nécessaires ou kits de diagnostic pour la mise en oeuvre desdits procédés.

Un premier rétrovirus, dénommé HIV-1, a été décrit dans la demande de Brevet européen N° 138 667 du 14 septembre 1984. Un second rétrovirus, dénommé HIV-2, a été décrit dans la demande de Brevet européen N° 239 425 du 22 janvier 1987.

Ces deux rétrovirus ont pour cibles préférentielles les lymphocytes T4 humains et présentent un effet cytopathogène à l'égard de ces lymphocytes.

Les lysats des virus HIV-1 contiennent des protéines du noyau dénommées protéines *gag* et *pol* qui présentent une homologie de 60 % avec les protéines *gag* et *pol* correspondantes des virus HIV-2, et des protéines de l'enveloppe dénommées protéines *env* qui présentent seulement une homologie de 40 % avec les protéines *env* correspondantes des virus HIV-2.

Ces lysats ont été utilisés dans des tests de diagnostic du SIDA. Afin d'augmenter la spécificité de ces tests, il a été proposé de substituer aux lysats viraux des produits qui ne proviennent pas de cellules infectées par le virus, comme des peptides synthétiques ou des peptides obtenus d'organismes recombinants.

Les études effectuées sur les différentes protéines des virus HIV-1 et HIV-2 ont permis de reconnaître des peptides ayant des séquences identiques ou proches des séquences obtenues dans les protéines *gag*, ou dans les protéines *env* de ces virus. De tels peptides, issus des protéines majeures du virus HIV-1, ont été décrits, notamment dans le Brevet américain N° 4 629 783. Des peptides, issus des protéines majeures du virus HIV-2 ont été décrits, notamment dans la demande de Brevet français N° 2 610 632.

On a également proposé à des fins diagnostic ou thérapeutique, des peptides issus des glycoprotéines gp41 et gp42 des virus HIV-1 et HIV-2, modifiés à leur extrémité, de façon à faciliter le couplage desdits peptides à des supports ou à des molécules porteuses.

Il a aussi été proposé dans la demande de Brevet européen N° 326 490, des peptides cyclisés par formation de ponts disulfure entre deux résidus cystéines.

La présente invention concerne de nouveaux peptides correspondant à la glycoprotéine d'enveloppe gp41 de HIV-1. Ces peptides peuvent notamment être utilisés chez l'Homme pour le diagnostic *in vitro* d'une infection par le virus HIV-1 et ils permettent une discrimination poussée entre les infections dues à des virus HIV-2 et celles dues à des virus HIV-1.

Un autre but de la présente invention est de fournir des peptides antigéniques dont la séquence en aminoacides est modifiée de façon à prévenir la formation de ponts disulfure non désirés.

Pour désigner ci-après les résidus d'aminoacides entrant dans la constitution des peptides selon l'invention, on aura recours aux normes IUPAC-IUB figurant dans Nucleic Acids Research 13, 3021-3030 (1985) et dans The Biochemical Journal, 219, N°2, 345-373 (1984).

Les peptides de l'invention correspondant à la glycoprotéine d'enveloppe de HIV-1 répondent à la formule suivante : 
X-Arg-Ile-Leu-Ala-Val-Glu-Arg-Tyr-Leu-Lys-Asp-Gln-Gln-
Leu-Leu-Gly-Ile-Trp-Gly-Cys-Ser-Gly-Lys-Leu-Ile-Cys-Z (I),
 dans laquelle X représente un groupe NH₂ libre ou amidé par un ou deux groupes alcoyle comprenant de 1 à 5 atomes de carbone et Z représente soit un groupe OH libre ou alcoxyle et contenant alors un groupe alcoyle comprenant de 1 à 5 atomes de carbone, et sont caractérisés en ce qu'au moins un des résidus Cys est délété, substitué ou protégé.

La présente invention concerne aussi de nouveaux peptides correspondant à la glycoprotéine d'enveloppe gp42 de HIV-2. Ces peptides peuvent notamment être utilisés pour le diagnostic *in vitro* chez l'Homme d'une infection par le virus HIV-2 et permettent une discrimination poussée entre les infections dues à des virus HIV-2 et celles dues à des virus HIV-1.

Comme pour les peptides dérivant de la gp41 de HIV-1, un autre but de la présente invention est de fournir des peptides antigéniques dont la séquence en aminoacides est modifiée de façon à prévenir la formation de ponts disulfure non désirés.

Ce but est atteint grâce aux peptides de l'invention correspondant à la glycoprotéine d'enveloppe de HIV-2 et répondant à la formule suivante : 
X-Ala-Ile-Glu-Lys-Tyr-Leu-Gln-Asp-Gln-Ala-Arg-Leu-Asn-
Ser-Trp-Gly-Cys-Ala-Phe-Arg-Gln-Val-Cys-Z   (II)
 dans laquelle X représente un groupe NH₂ libre ou amidé par un ou deux groupes alcoyle comprenant de 1 à 5 atomes de carbone et Z représente soit un groupe OH libre ou alcoxyle et contenant alors un groupe alcoyle comprenant de 1 à 5 atomes de carbone, et dans laquelle au moins un des résidus Cys est délété, substitué ou protégé.

La délétion, la substitution ou la protection d'au moins un des résidus Cys permet d'empêcher la formation de ponts disulfure, et donc la cyclisation ou la polymérisation des peptides. Il était nécessaire avec les peptides de l'Art antérieur d'utiliser des agents réducteurs afin de supprimer les ponts disulfure et ainsi d'éviter lors de la mise en oeuvre de ces peptides dans des procédés de diagnostic, la détection de faux positifs.

Par moyens de substitution ou de protection d'au moins un des résidus Cys, on préfère :
- la substitution d'au moins un des résidus Cys par un résidu Ser,
- la substitution d'au moins un des résidus Cys par un résidu Ala,
- la substitution d'au moins un des résidus Cys par un résidu acide gamma amino butyrique,
- la protection d'au moins un des résidus Cys par un groupement acétamidométhyle,
- la substitution des deux résidus Cys par un résidu Asp et un résidu Glu liés par un pont amide.

La substitution d'un résidu Cys par un résidu Ser, un résidu Ala ou un résidu acide gamma aminobutyrique, et la protection d'un résidu Cys par un groupement acétamidométhyle peuvent, bien entendu, être combinés dans les peptides de l'invention.

Les peptides selon l'invention peuvent être préparés par les techniques classiques de synthèse peptidique en phase solide, soit par condensation successive des résidus d'acides aminés dans l'ordre requis, soit par condensation des résidus d'acides aminés sur un fragment préalablement formé et contenant déjà plusieurs acides aminés dans l'ordre approprié ou encore par condensation de plusieurs fragments préalablement préparés, en prenant soin de protéger au préalable toutes les fonctions réactives portées par les résidus d'acides aminés ou les fragments, exceptés les fonctions amine et carboxyle engagées dans la liaison peptidique formée lors de la condensation.

Selon un exemple de préparation du peptide de formule (I), on fixe sur une résine, par l'intermédiaire de son groupe carboxylique, le premier résidu d'acide aminé dont la fonction amine est protégée par un groupe terbutyloxycarbonyl, puis après avoir déprotégé la fonction amine en lavant la résine avec de l'acide trifluoroacétique dans du dichlorométhane, on couple en présence de diméthylformamide le second résidu d'acide aminé Ile, dont la fonction amine est protégée comme précédemment ; on fixe ainsi les uns après les autres les résidus d'acides aminés. Après déprotection, la fonction amine du résidu N-terminal Arg peut par exemple être acétylée par action d'un excès d'anhydride acétique en présence de diisopropyléthylamine.

Les chaînes latérales des acides aminés trifonctionnels doivent être protégées notamment par les groupes suivants : le cyclohexyl pour l'acide glutamique, le benzyl pour la thréonine, le tosyl pour l'arginine, le paraméthylbenzyl pour la cystéine, le 2,6-dichlorobenzyl pour la tyrosine, le fluorényl-méthyloxycarbonyl ou le 2-chlorobenzyloxycarbonyl pour la lysine.

Après avoir éliminé tous les groupes protecteurs, le peptide selon l'invention est libéré du support solide. Le produit brut est lyophilisé et purifié par chromatographie en phase liquide à moyenne pression, permettant d'obtenir des peptides purs à environ 70% ; les produits sont alors purifiés par chromatographie en phase liquide à haute pression permettant d'obtenir des peptides purs à 95%.

Outre les peptides précités, l'invention concerne aussi les peptides dans lesquels la liaison peptidique (-CO-NH-) est remplacée par exemple par les structures suivantes :
-CO-N(CH₃)-, -CH₂-CH₂-, -CO-CH₂-
ou dans lesquels le squelette peptidique présente un ou plusieurs groupes intercalés tels que les groupes -CH₂-, NH-, -O-. La présente invention englobe également les peptides dans lesquels les résidus d'acides aminés présentant un carbone asymétrique sont sous forme D ou L.

L'invention concerne également une composition antigénique contenant au moins un des peptides de l'invention ou au moins un oligomère de ce peptide reconnaissant spécifiquement la présence d'anticorps anti-HIV-1 et/ou anti-HIV-2.

On peut réaliser l'oligomérisation par toute technique de polymérisation d'un peptide, la polymérisation étant effectuée jusqu'à l'obtention d'un oligomère contenant le nombre de monomères permettant d'obtenir l'immunogénicité souhaitée.

L'invention concerne encore une composition immunogène contenant au moins un des peptides de l'invention ou au moins un oligomère de ce peptide ou ce peptide conjugué à une molécule porteuse, en association avec un véhicule pharmaceutiquement acceptable, pour la préparation de vaccins induisant la production d'anticorps contre lesdits peptides en quantité suffisante pour inhiber efficacement le rétrovirus HIV-1 et/ou le rétrovirus HIV-2.

A titre d'exemple de molécules porteuses, on peut citer des protéines naturelles comme l'anatoxine tétanique, l'ovalbumine ou des sérums albumines.

Les peptides de l'invention possèdent des propriétés antigéniques et peuvent donc être utilisés dans des procédés de détection d'une infection par les virus HIV-1 et/ou HIV-2.

L'invention a donc également pour objet un procédé de diagnostic *in vitro* de l'infection par les virus HIV-1 et/ou HIV-2, dans un échantillon biologique tel que le sérum humain comprenant de manière générale:
- la mise en contact de cet échantillon biologique avec au moins un des peptides selon l'invention ou un conjugué de ce peptide avec une molécule porteuse ; et
- la détection de la présence éventuelle de complexes antigène-anticorps.

La détection des complexes antigènes-anticorps éventuellement formés est avantageusement effectuée par des tests immunoenzymatiques, immmunofluorescents, radioimmunologiques ou des tests de radioimmuno-précipitation.

L'invention concerne donc aussi les peptides précédents marqués notamment par un isotope radioactif, une enzyme, une substance fluorescente, une molécule chargée électriquement, une particule colorée, la biotine, un composé organométallique.

A titre d'exemple des procédés de détection des anticorps anti-HIV-1 et/ou anti-HIV-2 sus-mentionnés, on peut citer notamment un test immunoenzymatique indirect dans le sérum ou le plasma humain, comprenant les étapes suivantes :
- dépôt d'une quantité déterminée d'un peptide de l'invention, d'un mélange de peptide de l'invention ou d'une composition contenant lesdits peptides dans les puits d'une plaque de microtitration ;
- distribution des sérums ou plasma à étudier dilués, ainsi que des sérums de contrôle positifs et négatifs, dans lesdits puits ;
- incubation pendant une durée suffisante pour permettre la fixation des anticorps sériques éventuellement présents, aux peptides de l'invention spécifiques de HIV-1 et/ou aux peptides de l'invention spécifique de HIV-2 ;
- lavage de la microplaque ;
- introduction dans les puits d'un conjugué anti-IgG humaine marqué à la peroxydase, se liant aux immunoglobulines du sang retenues sur la phase solide ;
- élimination de la fraction non liée ;
- révélation de la présence éventuelle de l'enzyme par un substrat chromogène ;
- détection et comparaison avec les sérums de contrôle.

La délétion, la substitution ou la protection d'au moins un résidu Cys dans les peptides de l'invention, évite lors du dépôt en phase liquide du peptide sur les plaques de microtitration, la formation de ponts disulfure et donc la formation de polymère.

Parmi les procédés de détection des anticorps-anti-HIV-1 et/ou anti-HIV-2, selon l'invention, on peut citer en outre, un test sérologique immunoenzymatique indirect rapide comprenant les étapes suivantes :
- dépôt d'une quantité déterminée d'au moins un peptide de l'invention sur une membrane, saturée puis séchée ;
- filtration au travers de cette membrane d'un sérum à tester (ou plasma ou sang total) ; suivi du dépôt d'un conjugué (par exemple anti-IgG humain couplé à la peroxydase) ;
- après lavage, la réaction est révélée par dépôt d'un substrat chromogène de la peroxydase. Seuls les anticorps anti-HIV-1 et/ou anti-HIV-2 fixés aux peptides sont révélés dans cette réaction par un signal coloré. Un témoin interne de validation est inclus dans le test ;
- la présence d'un signal coloré en position du dépôt préliminaire du peptide ainsi que la présence du contrôle de validation interne permet l'interprétation positive (présence d'anticorps-anti-HIV-1 et/ou anti-HIV-2) pour l'échantillon considéré, en un temps de trois minutes.

L'invention concerne enfin des nécessaire ou kits pour le diagnostic *in vitro* de l'infection par le virus HIV-1 et/ou le virus HIV-2, dans un échantillon biologique comprenant :
- une quantité donnée d'au moins un peptide selon l'invention, ou un mélange de ces peptides, ou un conjugué de ce peptide avec une molécule porteuse ;
- au moins un réactif pour la constitution d'un milieu propice à la réalisation d'une réaction immunologique ;
- un ou plusieurs réactifs, éventuellement marqué, pour la détection des complexes antigène-anticorps formés lors de la réaction immunologique ;
- au moins un échantillon de contrôle dépourvu d'anticorps ou contenant une quantité connue d'anticorps reconnus par lesdits peptides.

Dans le cas d'une détection simultanée d'une infection par les virus HIV-1 et HIV-2, on peut avantageusement utiliser deux peptides, chacun spécifique de l'un des virus, marqués par des moyens différents afin de pouvoir discerner la présence d'anticorps anti-HIV-1 et anti-HIV-2.

Une étude comparative a été réalisée afin de montrer que les modifications effectuées au niveau des résidus Cys sur les peptides de formule (I) et (II) ne changent pas leur fonctionnalité antigénique.

### I - ETUDES DES PEPTIDES DERIVANT DE LA GLYCOPROTEINE D'ENVELOPPE DU VIRUS HIV-1

Cette étude a consisté à tester sur des sérums de réactivité connue, d'une part le peptide natif de formule : 
Arg-Ile-Leu-Ala-Val-Glu-Arg-Tyr-Leu-Lys-Asp-Gln-Gln-Leu-
Leu-Gly-Ile-Trp-Gly-Cys-Ser-Gly-Lys-Leu-Ile-Cys (I)
 et d'autre part, ce peptide dont les deux résidus Cys ont été substitués par deux résidus Ser.

Chacun des deux peptides a été utilisé dans un test de diagnostic sérologique sur plaque de microtitration, selon le même protocole suivant :

### Sensibilisation des plaques de microtitration :

- les peptides sont repris en milieu liquide. Après dilution en tampon à une concentration de 3 µg/ml, les cupules des microplaques sont sensibilisées par 100 µl de cette solution, 16 heures à 37°C ;
- les plaques sont ensuite vidées et 300 µl de solution de saturation sont ajoutés ; incubation 2 heures à température du laboratoire ;
- après quatre cycles de lavages, les plaques sont séchées à 37°C avant utilisation (stockage en sac étanche avec dessiccant).

### Réalisation de la réaction:

- après prélavage de la plaque de microtitration sensibilisée, 100 µl de l'échantillon (sérum de réactivité connue dilué au 1/25) sont déposés par cupule. Des témoins de manipulation sont inclus dans chaque série ; l'incubation est effectuée pendant 15 minutes à température ambiante.
- après un second lavage (4 cycles), afin d'éliminer les anticorps sériques non fixés, on distribue 100 µl d'anticorps anti-IgG humain couplé à la peroxydase (anticorps polyclonal d'origine caprine) ; l'incubation est effectuée 15 minutes à température ambiante ;
- un dernier cycle de lavage est ensuite effectué, et le conjugué peroxydase est révélé par 50 µl de substrat OPD (Ortho-phénylènediamine) en présence d'H₂O₂; cette incubation est conduite pendant 5 minutes à l'obscurité ;
- après apparition de la coloration, la réaction de chromogénèse est arrêtée par 100 µl d'HCl (1N). La densité optique (DO) est ensuite lue à 492 nm.

### Résultats:

Dans cette étude, l'antigénicité du peptide natif de formule (I) est comparée avec celle de sa forme substituée sérine (2 résidus Ser à la place de 2 résidus Cys). L'étude a porté sur les 18 sérums suivants, testés en double :
- 7 sérums anti-HIV-1 (dont 6 séroconversion);
- 8 négatifs;
- 2 anti-HIV-2;
- 1 anti-p24 isolé;
ainsi que sur les 3 témoins de manipulations : témoins négatif, anti-HIV-1 et anti-HIV-2.

Les résultats exprimés en DO sont rapportés dans le tableau I ci-après.

**TABLEAU I**

| NUMERO SERUM | REACTIVITE SERUM | PEPTIDE SUBSTITUE SERINE DO à 432nm en duplicate | PEPTIDE NATIF DE FORMUE (I) DO à 432 nm en duplicate |
|---|---|---|---|
| 1 | Anti-HIV-1 | 2,434 - 2,435 | 2,412 - 2,419 |
| 2 | Seroconversion HIV-1 | 1,235 - 1,213 | 0,585 - 0,599 |
| 3 | Seroconversion HIV-1 | 2,246 - 2,244 | 1,457 - 1,832 |
| 4 | Seroconversion HIV-1 | 1,888 - 1,969 | 1,122 - 0,851 |
| 5 | Seroconversion HIV-1 | 2,601 - 2,712 | 2,726 - 2,695 |
| 6 | Seroconversion HIV-1 | 1,023 - 1,022 | 1,520 - 1,520 |
| 7 | Seroconversion HIV-1 | 0,291 - 0,322 | 0,290 - 0,307 |
| 8 | Anti-HIV-2 | 0,825 - 0,800 | 0,421 - 0,487 |
| 9 | Anti-HIV-2 | 0,061 - 0,069 | 0,077 - 0,117 |
| 10 | Anti-P24 | 0,071 - 0,046 | 0,016 - 0,026 |
| 11 | Négatif | 0,053 - 0,052 | 0,052 - 0,054 |
| 12 | Négatif | 0,045 - 0,055 | 0,034 - 0,042 |
| 13 | Négatif | 0,044 - 0,037 | 0,051 - 0,043 |
| 14 | Négatif | 0,036 - 0,032 | 0,018 - 0,021 |
| 15 | Négatif | 0,068 - 0,065 | 0,050 - 0,060 |
| 16 | Négatif | 0,046 - 0,045 | 0,034 - 0,031 |
| 17 | Négatif | 0,019 - 0,014 | 0,004 - 0,006 |
| 18 | Négatif | 0,063 - 0,098 | 0,068 - 0,061 |
| Témoin négatif | Négatif | 0,023 - 0,026 | 0,012 - 0,014 |
| Témoin HIV-1 | Anti-HIV-1 | 1,354 - 1,346 | 1,296 - 1,305 |
| Témoin HIV-2 | Anti-HIV-2 | 0,024 - 0,018 | 0,018 - 0,021 |

### II - ETUDES DES PEPTIDES DERIVANT DE LA GLYCOPROTEINE D'ENVELOPPE DU VIRUS HIV-2.

Comme pour les peptides HIV-1, cette étude a consisté à tester sur des sérums de réactivité connue, le peptide natif de formule : 
Ala-Ile-Glu-Lys-Tyr-Leu-Gln-Asp-Gln-Ala-Arg-Leu-Asn-Ser-
Trp-Gly-Cys-Ala-Phe-Arg-Gln-Val-Cys  (II)
 ainsi que ce peptide dont les deux résidus Cys ont été, d'une part substitués par deux résidus Ser et d'autre part, liés par un pont disulfure.

Chacun des trois peptides a été utilisé dans un test de diagnostic sérologique sur plaque de microtitration, selon le même protocole suivant :

### Sensibilisation des plaques de microtitration:

- les peptides sont repris en milieu liquide. Après dilution en tampon à une concentration de 3 µg/ml, les cupules des microplaques sont sensibilisées par 100 µl de cette solution, 16 heures à 37°C.
- les plaques sont ensuite vidées et 300 µl de solution de saturation sont ajoutés ; incubation 2 heures à température du laboratoire ;
- après quatre cycles de lavages, les plaques sont séchées à 37°C, avant utilisation (stockage en sac étanche avec dessiccant) ;

### Réalisation de la réaction:

- après prélavage de la plaque de microtitration sensibilisée, 100 µl de l'échantillon (sérum de réactivité connue, dilué au 1/25) sont déposés par cupule. Des témoins de manipulation sont inclus dans chaque série ; l'incubation est effectuée pendant 15 minutes à température ambiante.
- après un second lavage (4 cycles), afin d'éliminer les anticorps sériques non fixés, on distribue 100 µl d'anticorps anti-IgG humain couplé à la peroxydase (anticorps polyclonal d'origine caprine) ; l'incubation est effectuée 15 minutes à température ambiante ;
- un dernier cycle de lavage est ensuite effectué, et le conjugué peroxydase est révélé par 50 µl de substrat OPD (Ortho-phénylènediamine) en présence d'H₂O₂; cette incubation est conduite pendant 5 minutes à l'obscurité ;
- après apparition de la coloration, la réaction de chromogénèse est arrêtée par 100 µl d'HCl (1N). La densité optique (DO) est ensuite lue à 492 nm.

### Résultats:

Dans cette étude, l'antigènicité du peptide natif de formule (II) est comparée avec celle de sa forme substituée sérine (2 résidus Ser à la place de 2 résidus Cys) ainsi qu'avec celle de sa forme cyclisée (2 résidus Cys liés par un pont disulfure). L'étude a porté sur les 13 sérums suivants testés en double :
- 6 sérums anti-HIV-2 ;
- 3 négatifs ;
- 2 anti-HIV-1;
- 2 anti-p24 isolé ;

ainsi que sur les 3 témoins de manipulations: témoins négatifs, anti-HIV-1 et anti-HIV-2.
Les résultats exprimés en DO sont rapportés dans le Tableau II ci-dessous.

**TABLEAU II**

| NUMERO SERUM | REACTITE SERUM | PEPTIDE NATIF DE FORMULE (II) DO à 432 nm en duplicate | PEPTIDE CYCLISE DO à 432 nm en duplicate | PEPTIDE SUBSTITUE SERINE DO à 432 nm en duplicate |
|---|---|---|---|---|
| 1 | Anti-HIV-2 | 2,672 - 2,682 | 2,691 - 2,717 | 2,681 - 2,691 |
| 2 | Anti-HIV-2 | 2,508 - 2,505 | 2,518 - 2,519 | 2,416 - 2,417 |
| 3 | Anti-HIV-2 | 2,399 - 2,372 | 2,383 - 2,399 | 2,328 - 2,313 |
| 4 | Anti-HIV-2 | 2,247 - 2,252 | 2,253 - 2,188 | 2,252 - 2,190 |
| 5 | Anti-HIV-2 | 1,951 - 1,956 | 1,911 - 1,949 | 1,762 - 1,781 |
| 6 | Anti-HIV-2 | 1,916 - 1,957 | 1,857 - 1,887 | 1,869 - 1,867 |
| 7 | Négatif | 0,051 - 0,109 | 0,054 - 0,062 | 0,059 - 0,057 |
| 8 | Négatif | 0,076 - 0,068 | 0,060 - 0,065 | 0,073 - 0,074 |
| 9 | Négatif | 0,042 - 0,042 | 0,041 - 0,043 | 0,040 - 0,043 |
| 10 | Anti-HIV-1 | 0,091 - 0,094 | 0,066 - 0,067 | 0,123 - 0,183 |
| 11 | Anti-HIV-1 | 0,148 - 0,143 | 0,092 - 0,089 | 0,066 - 0,125 |
| 12 | Anti-P24 | 0,076 - 0,063 | 0,053 - 0,55 | 0,063 - 0,055 |
| 13 | Anti-P24 | 0,064 - 0,073 | 0,061 - 0,069 | 0,056 - 0,051 |
| Témoin négatif | Négatif | 0,042 - 0,041 | 0,036 - 0,045 | 0,028 - 0,021 |
| Témoin HIV-1 | Anti-HIV-1 | 0,016 - 0,016 | 0,021 - 0,049 | 0,003 - 0,004 |
| Témoin HIV-2 | Anti-HIV-2 | 2,072 - 2,073 | 2,039 - 2,039 | 1,976 - 1,920 |

Le peptide de formule (II) cyclisé par un pont disulfure a été étudié à titre comparatif, mais ne fait pas partie des peptides de l'invention dont l'un au moins des résidus Cys est modifié de façon à éviter toute formation de ponts disulfure. Or, le pont disulfure du peptide cyclisé est une liaison faible qui peut être facilement cassée par oxygénation et donc autoriser une polymérisation néfaste à la qualité des tests.

Les modifications effectuées au niveau des résidus cystéines des peptides natifs de formules (I) et (II) ne changent pas leur fonctionnalité antigénique, car elles ne diminuent pas la réactivité ; une réactivité homologue est enregistrée avec des signaux parfois plus forts sur la forme substituée sérine.

En outre, ces modifications évitent l'éventuelle polymérisation du peptide (auto-polymérisation, cyclisation aléatoire par les cystéines) et permettent ainsi le mélange éventuel des peptides de l'invention avec d'autres peptides comprenant des groupements -SH libres, tels que les cystéines. Ces modifications augmentent également l'homogénéité de production lots à lots des peptides de l'invention.

## Revendications

1. Peptide dérivant de la glycoprotéine d'enveloppe de virus HIV et répondant à l'une des formules suivantes ; 
X-Arg-Ile-Leu-Ala-Val-Glu-Arg-Tyr-Leu-Lys-Asp-Gln-Gln-
Leu-Leu-Gly-Ile-Trp-Gly-Cys-Ser-Gly-Lys-Leu-Ile-Cys-Z (I)
X-Ala-Ile-Glu-Lys-Tyr-Leu-Gln-Asp-Gln-Ala-Arg-Leu-Asn-
Ser-Trp-Gly-Cys-Ala-Phe-Arg-Gln-Val-Cys-Z (II)
 dans lesquelles X représente un groupe NH₂ libre ou amidé par un ou deux groupes alcoyle comprenant de 1 à 5 atomes de carbone et Z représente soit un groupe OH libre ou alcoxyle et contenant alors un groupe alcoyle comprenant de 1 à 5 atomes de carbone, caractérisé en ce qu'au moins un des résidus Cys est délété, substitué ou protégé.

2. Peptide selon la revendication 1, caractérisé en ce qu'au moins un des résidus Cys est substitué par un résidu Ser.

3. Peptide selon la revendication 1, caractérisé en ce qu'au moins un des résidus Cys est substitué par un résidu Ala.

4. Peptide selon la revendication 1, caractérisé en ce qu'au moins un des résidus Cys est substitué par un résidu acide gamma amino butyrique.

5. Peptide selon la revendication 1, caractérisé en ce qu'au moins un des résidus Cys est protégé par un groupement acétamidométhyle.

6. Peptide selon la revendication 1, caractérisé en ce que les résidus Cys sont substitués par un résidu Asp et un résidu Glu liés par un pont amide.

7. Peptide selon les revendications 1 à 5, caractérisé en ce que les résidus Cys sont substitués et/ou protégés différemment.

8. Peptide selon l'une quelconque des Revendications 1 à 7, caractérisé en ce que, dans ledit peptide, la liaison peptidique est remplacée par une structure choisie parmi les structures suivantes :
-CO-N(CH₃)-, -CH₂-CH₂-, -CO-CH₂-.

9. Peptide selon l'une quelconque des Revendications 1 à 7, caractérisé en ce que le squelette peptidique présente un ou plusieurs groupes intercalés choisis parmi les groupes suivants : -CH₂-, -NH-, -O-.

10. Peptide selon l'une quelconque des Revendications précédentes, caractérisé en ce que les résidus d'acides aminés présentant un carbone asymétrique sont sous forme D ou L.

11. Composition antigénique contenant au moins un peptide selon l'une quelconque des revendications 1 à 10 ou au moins un oligomère de ce peptide, caractérisée en ce qu'elle reconnaît spécifiquement la présence d'anticorps anti-HIV-1 et/ou anti-HIV-2.

12. Composition immunogène contenant au moins un peptide selon l'une quelconque des revendications 1 à 10 ou au moins un oligomère de ce peptide conjugué à une molécule porteuse, en association avec un véhicule pharmaceutiquement acceptable pour la production de vaccins, caractérisée en ce qu'elle induit la production d'anticorps contre lesdits peptides en quantité suffisante pour inhiber efficacement les rétrovirus HIV-1 et/ou HIV-2.

13. Procédé de diagnostic *in vitro* de l'infection par les rétrovirus HIV-1 et/ou HIV-2, dans un échantillon biologique, caractérisé en ce qu'il comprend :
- la mise en contact de cet échantillon biologique avec au moins un peptide selon l'une des revendications 1 à 10 ou un conjugué de ce peptide avec une molécule porteuse ;
- la détection de la présence éventuelle de complexes antigène-anticorps.

14. Procédé de diagnostic *in vitro* de l'infection par le rétrovirus HIV-1 et/ou HIV-2, dans un échantillon biologique selon la revendication 13, caractérisé en ce que la détection des complexes antigène-anticorps éventuellement formés est effectuée par des tests immunoenzymatiques, immunofluorescents, radioimmunologiques ou des tests de radioimmuno-précipitation.

15. Nécessaire pour la mise en oeuvre d'un procédé de diagnostic *in vitro* de l'infection par les virus HIV-1 et/ou HIV-2, dans un échantillon biologique, caractérisé en ce qu'il comprend :
- une composition peptidique contenant au moins un peptide selon l'une des revendications 1 à 10, ou un conjugué de ce peptide avec une molécule porteuse ;
- au moins un réactif pour la constitution d'un milieu propice à la réalisation d'une réaction immunologique ;
- un ou plusieurs réactifs, éventuellement marqué pour la détection des complexes antigène-anticorps formés lors de la réaction immunologique ;
- au moins un échantillon de référence dépourvu d'anticorps contenant une quantité connue d'anticorps reconnus par ladite composition peptidique.

## Patentansprüche

1. Vom Hüllglykoprotein des HIV-Virus abgeleitetes Peptid einer der folgenden Formeln: 
X-Arg-Ile-Leu-Ala-Val-Glu-Arg-Tyr-Leu-Lys-Asp-Gln-Gln-
Leu-Leu-Gly-Ile-Trp-Gly-Cys-Ser-Gly-Lys-Leu-Ile-Cys-Z (I)
 
X-Ala-Ile-Glu-Lys-Tyr-Leu-Gln-Asp-Gln-Ala-Arg-Leu-Asn-
Ser-Trp-Gly-Cys-Ala-Phe-Arg-Gln-Val-Cys-Z (II)
 in denen X für eine freie oder mit einer oder zwei Alkylgruppen mit 1 bis 5 Kohlenstoffatomen amidierte NH₂-Gruppe steht, und Z entweder für eine freie OH-Gruppe oder eine Alkoxylgruppe steht und dann eine Alkylgruppe mit 1 bis 5 Kohlenstoffatomen enthält, dadurch **gekennzeichnet,** daß wenigstens einer der Cys-Reste deletiert, substituiert oder geschützt ist.

2. Peptid nach Anspruch 1, dadurch **gekennzeichnet,** daß wenigstens einer der Cys-Reste durch einen Ser-Rest substituiert ist.

3. Peptid nach Anspruch 1, dadurch **gekennzeichnet,** daß wenigstens einer der Cys-Reste durch einen Ala-Rest substituiert ist.

4. Peptid nach Anspruch 1, dadurch **gekennzeichnet,** daß wenigstens einer der Cys-Reste durch einen y-Aminobuttersäurerest substituiert ist.

5. Peptid nach Anspruch 1, dadurch **gekennzeichnet,** daß wenigstens einer der Cys-Reste durch eine Acetamidomethylgruppierung geschützt ist.

6. Peptid nach Anspruch 1, dadurch **gekennzeichnet,** daß die Cys-Reste durch einen Asp-Rest und einen Glu-Rest, die durch ein Brückenamid verbunden sind, substituiert sind.

7. Peptid nach den Ansprüchen 1 bis 5, dadurch **gekennzeichnet,** daß die Cys-Reste unterschiedlich substituiert und/oder geschützt sind.

8. Peptid nach einem der Ansprüche 1 bis 7, dadurch **gekennzeichnet,** daß in diesem Peptid die Peptidbindung durch eine Struktur ersetzt ist, die aus den folgenden Strukturen ausgewählt ist:
-CO-N(CH₃)-, -CH₂-CH₂-, -CO-CH₂-.

9. Peptid nach einem der Ansprüche 1 bis 7, dadurch **gekennzeichnet,** daß im Peptidskelett eine oder mehrere eingefügte Gruppen vorhanden sind, die aus den folgenden Gruppen ausgewählt sind: -CH₂-, -NH-, -O-.

10. Peptid nach einem der vorangegangenen Ansprüche, dadurch **gekennzeichnet,** daß die Aminosäurereste, die ein asymmetrisches Kohlenstoffatom enthalten, in der D- oder L-Form vorliegen.

11. Antigene Zusammensetzung, enthaltend wenigstens ein Peptid nach einem der Ansprüche 1 bis 10 oder wenigstens ein Oligomeres dieses Peptids, dadurch **gekennzeichnet,** daß sie spezifisch das Vorhandensein von anti-HIV-1- und/oder anti-HIV-2-Antikörpern erkennt.

12. Immunogene Zusammensetzung, enthaltend wenigstens ein Peptid nach einem der Ansprüche 1 bis 10 oder wenigstens ein mit einem Trägermolekül konjugiertes Oligomeres dieses Peptids, in Verbindung mit einem für die Herstellung von Impfstoffen annehmbaren pharmazeutischen Träger, dadurch **gekennzeichnet,** daß sie die Bildung von Antikörpern gegen die genannten Peptide in einer Menge induziert, die ausreichend ist, um in effektiver Weise die Retroviren HIV-1 und/oder HIV-2 zu inhibieren.

13. Verfahren zur in-vitro-Diagnostik der Infektion mit den Retroviren HIV-1 und/oder HIV-2 in einer biologischen Probe, dadurch **gekennzeichnet,** daß es die folgenden Stufen umfaßt:
- Inkontaktbringen dieser biologischen Probe mit wenigstens einem Peptid nach einem der Ansprüche 1 bis 10 oder einem Konjugat dieses Peptids mit einem Trägermolekül;
- Nachweis von unter Umständen vorhandenen Antigen-Antikörper-Komplexen.

14. Verfahren zur in-vitro-Diagnostik von Infektionen mit dem Retrovirus HIV-1 und/oder HIV-2 in einer biologischen Probe, nach Anspruch 13, dadurch **gekennzeichnet,** daß der Nachweis der unter Umständen gebildeten Antigen-Antikörper-Komplexe mit immunenzymatischen Tests, Immunfluoreszenstests, radioimmunologischen Tests oder mit Radioimmunpräzipitationstests durchgeführt wird.

15. Kit zur Durchführung des Verfahrens zur in-vitro-Diagnostik der Infektion mit dem HIV-1- und/oder HIV-2-Virus in einer biologischen Probe, dadurch **gekennzeichnet,** daß er:
- eine Peptidzusammensetzung, enthaltend wenigstens ein Peptid nach einem der Ansprüche 1 bis 10 oder ein Konjugat dieses Peptids mit einem Trägermolekül;
- wenigstens ein Reagens zur Erzeugung eines für die Durchführung einer immunologischen Reaktion günstigen Mediums;
- ein oder mehrere Reagens (Reagentien), das/die gegebenenfalls zum Nachweis von Antigen-Antikörper-Komplexen, die als Folge der immunologischen Reaktion gebildet werden, markiert ist/sind;
- wengistens eine Referenzprobe ohne Antikörper, die eine bekannte Menge von Antikörpern enthält, die von der Peptidzusammensetzung erkannt werden,
umfaßt.

## Claims

1. Peptide deriving from the HIV virus enveloping glycoprotein and complying with one of the following formulae: 
X-Arg-Ile-Leu-Ala-Val-Glu-Arg-Tyr-Leu-Lys-Asp-Gln-Gln-Leu-Leu-Gly-Ile-Trp-Gly-
Cys-Ser-Gly-Lys-Leu-Ile-Cys-Z (I)
 
X-Ala-Ile-Glu-Lys-Tyr-Leu-Gln-Asp-Gln-Ala-Arg-Leu-Asn-Ser-Trp-Gly-Cys-Ala-Phe-
Arg-Gln-Val-Cys-Z (II)
 in which X represents a free NH₂ group or a group anodised by one or two alkyl groups comprising from 1 to 5 carbon atoms and Z represents either a free OH group or alkoxyl group and then containing an alkyl group comprising from 1 to 5 carbon atoms, characterised in that at least one of the Cys residues is deleted, substituted or protected.

2. A peptide according to claim 1, characterised in that at least one of the Cys residues is substituted by a Ser residue.

3. A peptide according to claim 1, characterised in that at least one of the Cys residues is substituted by an Ala residue.

4. A peptide according to claim 1, characterised in that at least one of the Cys residues is substituted by a butyric amino gamma acid residue.

5. A peptide according to claim 1, characterised in that at least one of the Cys residues is protected by an acetamidomethyl group.

6. A peptide according to claim 1, characterised in that the Cys residues are substituted by an Asp residue and a Glu residue bonded by an amide bridge.

7. A peptide according to claims 1 to 5, characterised in that the Cys residues are differently substituted and/or protected.

8. A peptide according to any one of claims 1 to 7, characterised in that in the said peptide the peptide bond is replaced by a structure chosen from among the following structures:
-CO-N(CH₃)-, -CH₂-CH₂-, -CO-CH₂-.

9. A peptide according to any one of claims 1 to 7, characterised in that the peptide skeleton comprises one or a plurality of interposed groups chosen from the following groups: -CH₂-, -NH-, -O-.

10. A peptide according to any one of the preceding claims, characterised in that the amino acid residues which have an asymmetrical carbon are in D or L form.

11. An antigenic composition containing at least one peptide according to any one of claims 1 to 10 or at least one oligomer of this peptide, characterised in that it specifically recognises the presence of anti-HIV-1 and/or anti-HIV-2 antibodies.

12. An immunogenic composition containing at least one peptide according to any one of claims 1 to 10 or at least one oligomer of this peptide conjugated with a carrier molecule in association with a vehicle which is pharmaceutically acceptable for the production of vaccines, characterised in that it induces the production of antibodies against the said peptides in a sufficient quantity in order effectively to inhibit the HIV-1 and/or HIV-2 retroviruses.

13. A process for the *in vitro* diagnosis of infection by HIV-1 or HIV-2 retroviruses in a biological specimen, characterised in that it comprises:
- the bringing of this biological specimen into contact with at least one peptide according to one of claims 1 to 10 or a form of this peptide which is conjugated with a carrier molecule;
- detection of the possible presence of antigen-antibody complexes.

14. A process for the *in vitro* diagnosis of infection by HIV-1 and/or HIV-2 retroviruses in a biological specimen according to claim 13, characterised in that the detection of any antigen-antibody complexes which may have formed is carried out by immuno-enzymatic, immuno-fluorescent, radio-immunological tests or radio-immunoprecipitation tests.

15. A kit for carrying out a process for the *in vitro* diagnosis of infection by HIV-1 and/or HIV-2 viruses in a biological specimen, characterised in that it comprises:
- a peptide composition containing at least one peptide according to one of claims 1 to 10 or a form of this peptide in which it is conjugated with a carrier molecule;
- at least one reagent for the constitution of a medium conducive to the carrying out of an immunological reaction;
- one or a plurality of reagents possibly marked for the detection of antigen-antibody complexes formed during the immunological reaction;
- at least one reference specimen which is bereft of antibodies containing a known quantity of antibodies recognised by the said peptide composition.
